# EUROPEAN PATENT SPECIFICATION

(11) **EP 2 119 402 B1**
(45) Date of publication and mention of the grant of the patent: **01.08.2012**
(21) Application number: 09006594.7
(22) Date of filing: 15.05.2009
(51) Int. Cl.: A61B 17/29, A61B 1/018, A61B 17/00, A61B 19/00

(54) **Endoscopic apparatus**
Endoskopievorrichtung
Appareil d'endoscopie

(30) Priority: 15.05.2008 US 120832
(43) Date of publication of application: 18.11.2009
(73) Proprietor: OLYMPUS MEDICAL SYSTEMS CORP., Tokyo 151-0072 (JP)
(72) Inventor: Yamatani, Ken, Tokyo 151-0072 (JP)
(74) Representative: von Hellfeld, Axel

(56) References cited:
- US-A- 5 984 932
- US-A1- 2004 138 525
- US-A1- 2005 085 691
- US-A1- 2008 051 631

## Description

### BACKGROUND OF THE INVENTION

### Field of the Invention

The present invention relates to an endoscopic apparatus that is, for example, combined with a flexible endoscope and used by insertion into a body cavity.

### Description of Related Art

Heretofore, endoscopic apparatuses have been used in order to observe and treat a diseased portion or the like in a body cavity of an examinee. For example, this endoscopic apparatus has been known which connects an elongate flexible inserting portion inserted into a body cavity from the distal side with an operating portion which operates the inserting portion.

An observation body for observing in a frontal direction, and a distal-end configuration portion with two arm portions on the distal end face which are provided with a treatment tool inserted therein, the treatment tool being capable of conducting a treatment, are provided at the distal end portion of the inserting portion. A tubular bending portion which can be bent is connected to the proximal side of the distal-end configuration portion, and in addition, a flexible tubular portion, which is flexible and connected to the operating portion, is connected to the proximal side of the bending portion. The distal end portion of an operating wire which communicates the bending portion with the interior of the flexible tubular portion is fixed on the proximal side of the distal-end configuration portion, and the proximal end portion of the operating wire is provided in the operating portion and attached to an angle knob with which the operating wire can be pulled.

Furthermore, working channels are formed from the distal end portions of the two arm portions to forceps stoppers provided in the operating portion via the inserting portion respectively. A treatment can be conducted by inserting the treatment tool in the working channel and protruding the distal end portion of the treatment tool from the distal end of the respective arm portions.

In the case of the endoscopic apparatus configured as above, when the inserting portion is inserted into a body cavity of an examinee, the insertion is conducted as the surroundings are observed by the observation body and the bending portion is bent with the angle knob in a state such that the distal end portions of the treatment tools are not protruded from the distal end portions of the two arm portions. In addition, the inserting portion is fixed in a state such that the two arm portions are directed toward the diseased portion, and a treatment is conducted while protruding the distal end portions of the treatment tools from the distal end portions of the arm portions respectively.

Here, in the previous endoscopic apparatus described above, as the two arm portions provided on the distal end face of the distal-end configuration portion have been protruded to the frontal direction in order to make the outer diameter of the inserting portion containing the two arm portions smaller when the inserting portion is inserted into a body cavity, there has been a problem that it is difficult to observe the surroundings because the sight of the observation body at the insertion is obstructed by the proximal side of the two arm portions provided just next to the observation body. Additionally, the obstruction of the proximal side of the two arm portions into the sight region of the observation body has made the treatment difficult also when a treatment of a diseased portion is conducted.

US 2004/0138525 A1 discloses an endoscope comprising an elongate deflectable shaft which receives two tool arms. The tool arms protrude from a distal end of the shaft and are accommodated within arm guide lumens extending through a central lumen of the shaft. A scope lumen is disposed between the arm guide lumens to facilitate viewing of the tool arms. The two-part form of claim 1 is based on this document.

US 2008/0051631 A1 relates to an endoscope, wherein two tool arms protrude from a distal end of a sheath which, in addition to the tool arms, receives an objective lens and two illuminating lenses.

US 2005/0085691 A1 describes an endoscope comprising an insertion member being provided with working segments. The working segments are bound in a cylindrical configuration about a visualization segment by a slidable sheath.

### SUMMARY OF THE INVENTION

The present invention has been made in light of the foregoing circumstances, and its object is to offer an endoscopic apparatus increasing frontal visibility during insertion and the visibility of the distal end portion of the arm portion during the treatment of a diseased portion.

An endoscope according to the present invention comprises the features defined in claim 1.

The present invention is an endoscopic apparatus which is provided with an elongate tubular inserting portion; a plurality of arm portions provided on a distal end face of the inserting portion and protruded to the frontal direction which are provided with treatment tools inserted therein, the treatment tools being capable of conducting a treatment; a cylindrical sheath which covers a circumferential surface of the inserting portion, which is movable to a distal side and a proximal side, and which supports the distal side of the plurality of the arm portions when moving to the distal side; and an observation body which is attached inside a distal end portion of the sheath, and by which a frontal view is observed.

### BRIEF DESCRIPTION OF THE DRAWINGS

FIG. 1 is a whole view of an endoscopic
FIG. 2 is a whole view of the endoscope for treatment equipped with the endoscopic apparatus.
FIG. 3 is a perspective view as seen from the A direction in FIG. 1.
FIG. 4 shows the constitution during the insertion of the inserting portion of the endoscopic apparatus into a body cavity.
FIG. 5 is a cross-sectional view of the constitution during the insertion of the inserting portion of the endoscopic apparatus into a body cavity.
FIG. 6 shows the constitution during the treatment of the diseased portion with the inserting portion of the endoscopic apparatus.
FIG 7 is a cross-sectional view of the constitution during the insertion of the inserting portion of a further endoscopic apparatus into a body cavity.
FIG. 8 shows the constitution during the treatment of the diseased portion with the inserting portion of the endoscopic apparatus.
FIG. 9 is a plan view during the insertion of the inserting portion of a further endoscopic apparatus into a body cavity.
FIG. 10 is a side view during the insertion of the inserting portion of the endoscopic apparatus into a body cavity.
FIG. 11 is a plan view during the treatment of the diseased portion with the inserting portion of the endoscopic apparatus.
FIG 12 shows a first modification of the endoscopic apparatus.
FIG. 13 shows a second modification of the endoscopic apparatus.
FIG. 14 is a partial cross-sectional view during the insertion of the inserting portion of the endoscopic apparatus of the present invention into a body cavity.
FIG. 15 is a plan view during the treatment of the diseased portion with the inserting portion of the endoscopic apparatus of the present invention.
FIG 16 is a plan view of the constitution during the treatment of the diseased portion with a further endoscopic apparatus.
FIG. 17 is a plan view of the constitution during the treatment of the diseased portion with a first modification of the endoscopic apparatus.
FIG 18 is a plan view of the constitution during the treatment of the diseased portion with a second modification of the endoscopic apparatus.

### PREFERRED EMBODIMENTS

Hereibelow, the respective embodiments of the present invention shall be described.

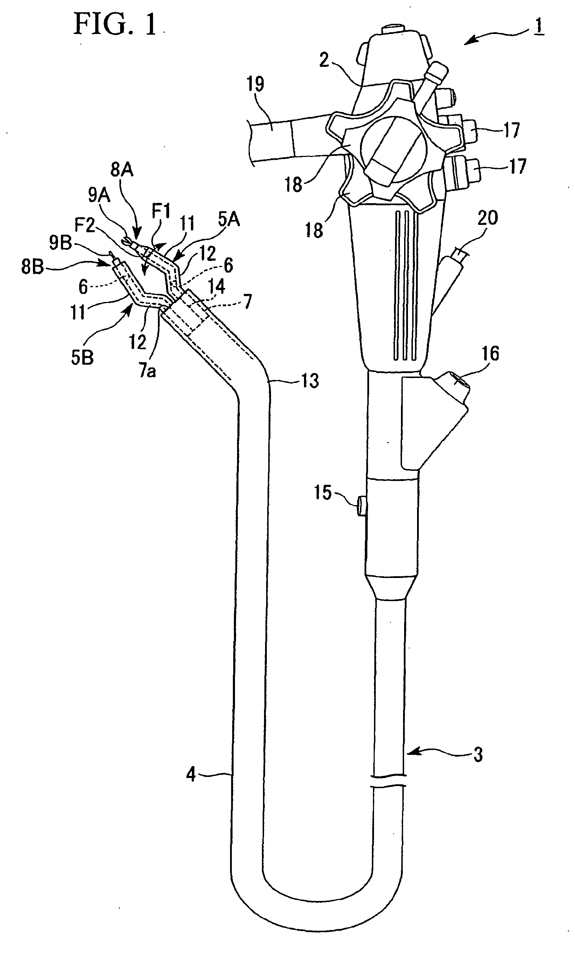

As shown in FIG 1, an endoscopic apparatus 1 is unified and provided with a tubular inserting portion 3 from one end of an operating portion 2. The inserting portion 3 is elongate and flexible. That is, the inserting portion 3 has a cylindrical sheath 4, with flexibility, covers the circumferential surface of the inserting portion 3 and is capable of moving to the distal side and proximal side, the first and second arm portions 5A, 5B which are capable of bending are protruded in a frontal direction and provided on the distal end face 7a of the distal-end constitution portion 7 provided at the distal end portion of the inserting portion 3. Working channels 6 are formed inside the respective arm portions 5A, 5B, and communicate to a connecting sheath 20 described later via the inserting portion 3 and the operating portion 2. Treatment tools 8A, 8B are inserted in the working channels 6 respectively, and treatment portions 9A, 9B of the treatment tools 8A, 8B are protruded from the distal end portions of the arm portions 5A, 5B respectively. The treatment tools 8A, 8B enable the first and second arm portions 5A, 5B to perform treatment in a body cavity or the like.

The first bending portion 11 and the second bending portion 12 are formed in order from the distal side in each arm portion 5A, 5B, and enable the bending operation in a body by cooperating with the third bending portion 13 formed in the inserting portion 3.

In addition, an observation body 14 for observing in a frontal direction is attached inside the distal end portion of the sheath 4.

The first and second arm portions 5A, 5B may be inserted in other sheath protruded from the distal end of the sheath 4.

A forceps stopper 16 is provided on the side of one distal end portion of the operating portion 2 connecting to the inserting portion 3. The forceps stopper 16 communicates with the working channel 6 formed in the sheath 4. If other treatment tool not illustrated is inserted from here, the other treatment tool can also be protruded from the distal end of the first and second arm portions 5A, 5B. Besides this, a sheath operating lever 15, a switch 17, an angle knob 18, a controller not illustrated, and a universal cable 19 connected to a monitor are also provided with the operating portion 2. When the sheath operating lever 15 is moved forward and backward, it is configured so as to move the sheath 4 and the observation body 14 to the axial direction of the inserting portion 3. The switch 17 is operated, for example, when the air or water is sent or suctioned through the channel formed in the sheath 4. The angle knob 18 is used when the third bending portion 13 is bent to two directions relative to the axis. In addition, an image observed in the observation body 14 is sent to the monitor via the universal cable 19.

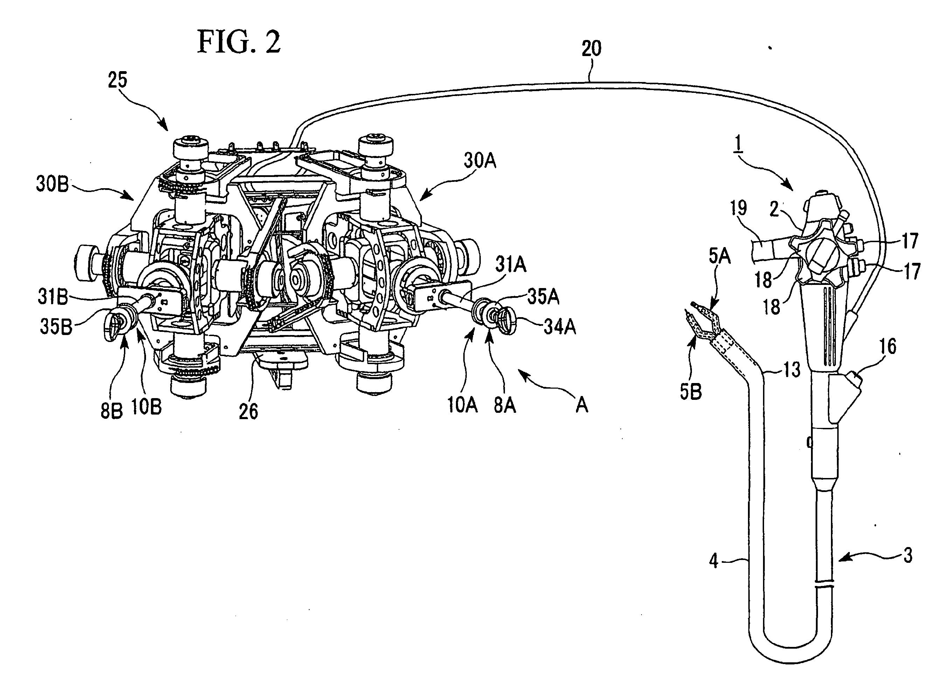

Furthermore, as shown in FIG. 2, the connecting sheath 20 which is elongate and flexible is provided from the other distal end portion of the operating portion 2, and an operating portion 25 is provided at the distal end portion of the connecting sheath 20.

The operating portion 25 has a base 26 which fixes the connecting sheath 20, and a first operating unit 30A and a second operating unit 30B are attached to the base 26. The first operating unit 30A has an operating stick 31 A in which an operating portion 10A of the treatment tool 8A is inserted, the treatment tool 8A being inserted through the first arm portion 5A. The operating portion 10A is supported via the operating stick 31A so as to move freely to the axial direction forward and backward and to tilt freely to the two directions to regard the axis as a center. The second operating unit 30B has an operating stick 31B in which an operating portion 10B of the treatment tool 8B in inserted, the treatment tool 8B being inserted through the second arm portion 5B. The operating portion 10B is supported via the operating stick 31B so as to move freely to the axial direction forward and backward and to tilt freely to the two directions to regard the axis as a center.

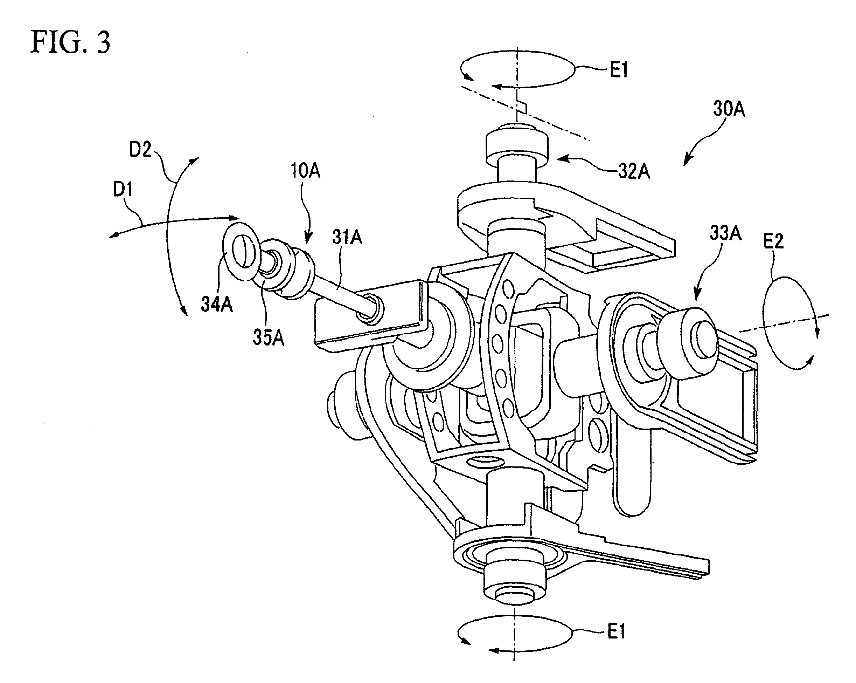

In the known constitution shown in FIG. 3, when an operator rotates the operating stick 31A to the direction D1, a first rotating mechanism 32A rotates to the direction E1, thereby the first bending portion 11 of the first arm portion 5A is bent to the direction F1 by a not-illustrated operating wire which is wound to the first rotating mechanism 32A as shown in FIG. 1. In addition, when an operator rotates the operating stick 31A to the direction D2, a second rotating mechanism 33A rotates to the direction E2, thereby the first bending portion 11 of the first arm portion 5A is bent to the direction F2 which is orthogonal to the direction F1 (the vertical direction with respect to the page of FIG. 3) by a not-illustrated operating wire which is wound to the second rotating mechanism

### 33A.

When the operating stick 31 B is rotated, the first bending portion 11 of the second arm portion 5B is bent in the same way. The detail descriptions are eliminated.

In addition, when the operating lever not illustrated is pushed out, the second bending portions 12 of the first and second arm portions 5A, 5B are both linear. However, when the operating lever is pulled and fixed, as shown in FIG. 1, the second bending portions 12 of the first and second arm portions 5A, 5B are maintained in a mutually separated and bent shape.

A holding forceps is used as the treatment tool 8A and a syringe is used as the treatment tool 8B in this embodiment. The operation to open and close the distal end portion of this holding forceps is conducted by hooking the fingers to the ring 34A and slider 35A, moving the slider 35A axially relative to the ring 34A, and pulling and pushing the not-illustrated operating wire connected to the treatment portion 9A as shown in FIG. 3. On the other hand, when an injection is conducted to a tissue by the syringe of the treatment portion 9B, the slider 35B provided in the second operating unit 30B is operated in the same way as shown in FIG. 2.

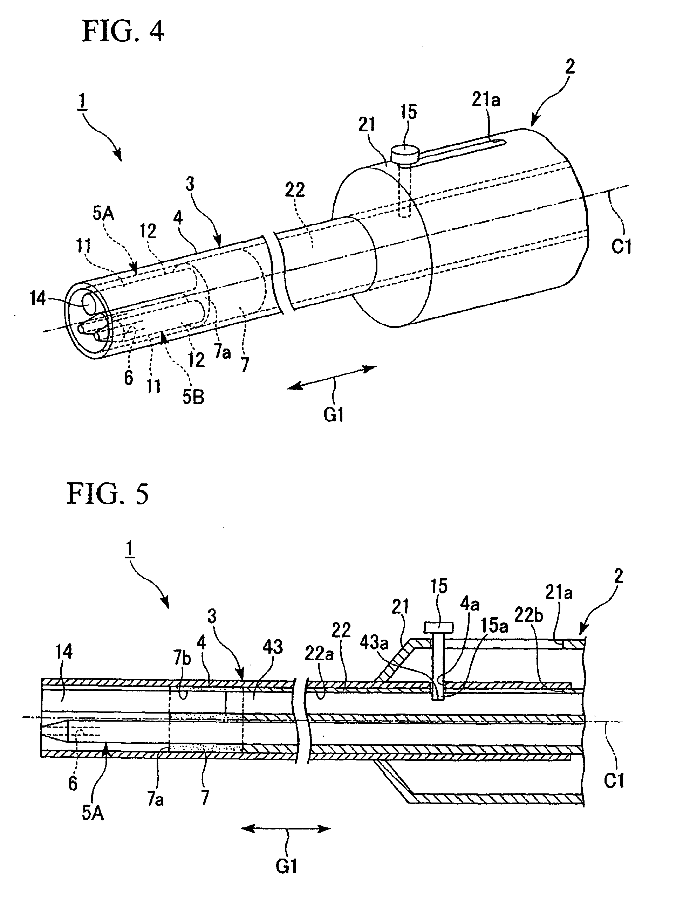

Next, the constitution of the inserting portion 3 at the insertion into a body cavity is shown in FIG. 4 and FIG. 5. These figures are different from the constitution shown in FIG 1 in a state such that the first and second arm portions 5A, 5B are parallel to each other and the treatment portions 9A, 9B are not protruded from the distal end portion of each arm portion 5A, 5B.

A cylindrical inserting-portion inner guide 22 which can be bent is connected to the proximal end portion of the distal-end constitution portion 7, and the proximal end portion of the inserting-portion inner guide 22 is connected to the operating portion 2. Instead of the inserting-portion inner guide 22, a bending piece which is connected in a freely rotating manner by a rivet or the like for bending the inserting portion 3 or a coil sheath may be used.

A guide hole 7b and a guide hole 22a extending to the axis direction G1 which is the direction of the axis C1 of the inserting portion 3 are formed respectively in the distal-end constitution portion 7 and the inserting-portion inner guide 22, and are communicated with each other. Light detecting elements such as a lens, a CCD and the like are provided in the observation body 14, and an observation cable 43 with which an image captured by the observation body 14 is transported to the monitor is connected. The observation body 14 and observation cable 43 are inserted through the guide hole 7b and guide hole 22a.

In addition, an operating-lever cover 21 which is cylindrical and has a bottom is attached along the axis direction G1 of the inserting portion 3 between the proximal end portion of the inserting portion 3 and the operating portion 2 so as to cover the sheath 4 from the outside. As mentioned below, to decrease the friction when the sheath 4 moves to the axial direction G1, it is preferable that lubricant or the like is coated on the circumferential surfaces of the distal-end constitution portion 7 and inserting-portion inner guide 22, and the portion where the internal surface of the operating-lever cover 21 contacts with the sheath 4. In the same way, to decrease the friction when the observation body 14 and the observation cable 43 moves to the axial direction G1, it is preferable that lubricant or the like is coated on the respective circumferential surfaces of the observation body 14 and the observation cable 43 as well.

A long-hole portion 21a is formed along the axial direction G1 on the circumferential surface of the operating-lever cover 21. A long-hole portion 22b is formed in the inserting-portion inner guide 22 at the face position to the long-hole portion 21 a of the operating-lever cover 21, and is communicated with the guide hole 22a. In addition, a circular hole portion 4a is formed in the sheath 4 at the face position to the distal side of the long-hole portion 21a, and a concave portion 43a which is reentrant to the radial direction is formed in the observation cable 43 at the face position of the long-hole portion 21a.

A rod-like sheath-operating lever 15 is engaged with the long-hole portion 21 a of the operating-lever cover 21, and can be moved to the axial direction G1 and radial direction without changing orientations by a not-illustrated positioning mechanism attached to the operating-lever cover 21

Next, a method of treating a diseased portion by the endoscopic apparatus 1 as constituted above shall be described.

First, in the operating portion 2, the operating portion 25 is made in a state such that the first and second arm portions 5A, 5B are parallel to each other by pushing the operating lever, and the treatment portions 9A, 9B are not protruded from the distal end portion of each arm portion 5A, 5B by pulling the operating portion 10A, 10B forward relative to operating sticks 31A, 31B respectively as shown in FIG. 4. In addition, the sheath-operating lever 15 is made to move to the distal side while being pushed. Here, when the sheath-operating lever 15 is pushed, the sheath-operating lever 15 is engaged with the concave portion 43a of the observation cable 43 through the proximal side of the long-hole portion 21a of the operating-lever cover 21, the circular-hole portion 4a of the sheath 4, and the proximal side of the long-hole portion 22b of the inserting-portion inner guide 22. Therefore, members of which the sheath-operating lever 15 is inserted through the long-hole portion when the sheath-operating lever 15 is moved to the distal side are not moved, and the sheath 4 and the observation body 14 are moved to the distal side. Thus, the cylindrical sheath 4 holds the distal side of each arm portion 5A, 5B.

Next, the inserting portion 3 is inserted into the body cavity of the examinee while the surroundings are observed by the observation body 14 and the first bending portion 11 of each arm portion 5A, 5B are bent by the first operating unit 30A and second operating unit 30B and the third bending portion 13 is bent by the angle knob 18 respectively.

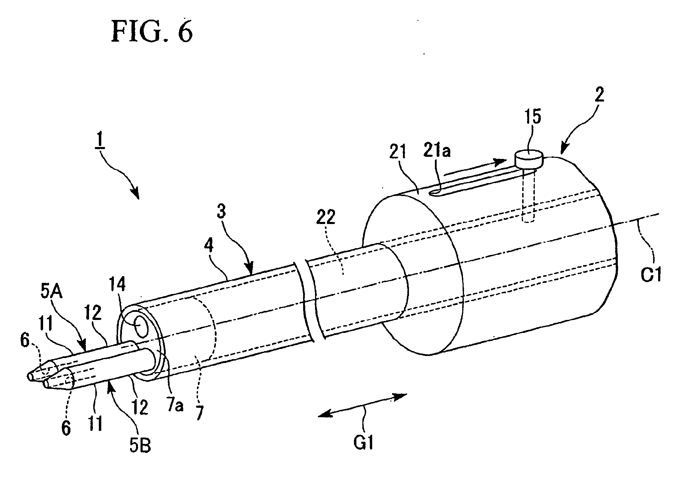

Next, the inserting portion 3 is fixed in a state such that the distal end portions of the two arm portions 5A, 5B direct toward the diseased portion, and the sheath operating lever 15 is moved to the proximal side while being pushed as shown in FIG. 6. Thus, the observation cable 43 of which the sheath-operating lever 15 is engaged with the concave portion 43a, and the sheath 4 of which the sheath-operating lever 15 is inserted through the circular-hole portion 4a move to the proximal side together with the sheath-operating lever 15, and each arm portion 5A, 5B has become in a state of protruding to the frontal direction from the distal end face 7a of the distal-end constitution portion 7. Furthermore, the treatment portions 9A, 9B of the treatment tools 8A, 8B are made protrude from the distal end portion of each arm portion 5A, 5B as shown in FIG. 1 by pushing the operating portion 10A, 10B relative to operating sticks 31A, 31B respectively. Moreover, the second bending portion 12 is fixed in a state to be bent so that the first and the second arm portions 5A, 5B separate from each other by pulling and fixing the operating lever.

Under this condition, while the diseased portion is observed by the observation body 14, the operating stick 31A is rotated and the slider 35A is moved while the first bending portion 11 of the first arm portion 5A is bent, and thereby the diseased portion is held by the treatment portion 9A. In addition, the diseased portion is punctured with the needle-like treatment portion 9B while bending the first bending portion 11 of the second arm portion 5B by rotating the operating stick 31B, not-illustrated liquid medicine or the like is poured into the diseased portion by moving the slider 35B.

As mentioned above, according to the endoscopic apparatus 1 of the present embodiment, the visibility in the frontal direction with the observation body 14 is increased by moving the sheath 4 and the observation body 14 to the distal side when the inserting portion 3 is inserted into the body cavity and it is possible to prevent each arm portion 5A, 5B from damaging the circumferential tissue by holding the distal sides of the first and the second arm portions with the sheath 4.

In addition, the distal side of the sheath 4 which covers the distal side of each arm portion 5A, 5B can also be bent by bending the first bending portion 11 of each arm portion 5A, 5B at the insertion, and thereby the distal side of the sheath can be bent more freely. Therefore, it is possible to make the distal end of the inserting portion 3 reach the diseased portion in a shorter amount of time.

In the present example, only the sheath 4 can be moved in the axial direction G1 by moving the sheath-operating lever 15 to the axial direction G1 in a state such that the sheath-operating lever 15 is not pushed out and inserted only through the circular-hole portion 4a of the sheath 4.

Next, the second example of an endoscope apparatus shall be described, and portions that are common with the abovementioned first example shall be designated by the same reference numbers, descriptions thereof shall be omitted, and only the differences shall be described.

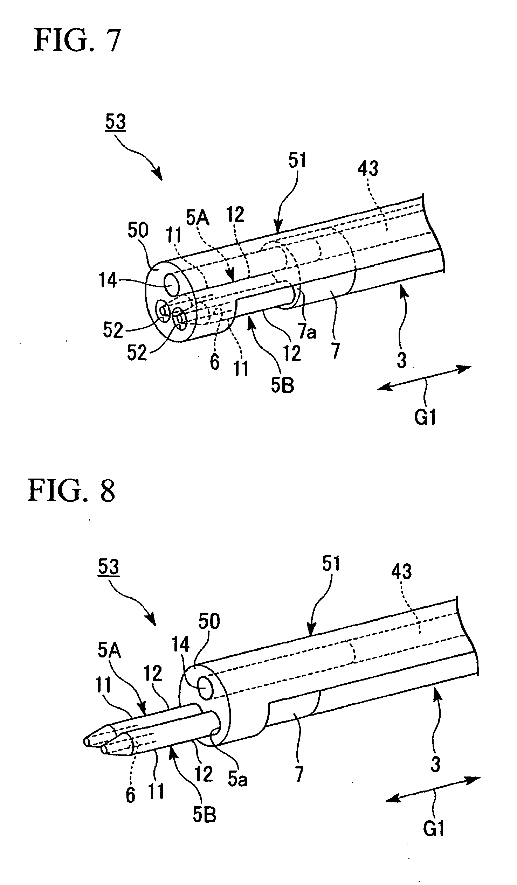

In this example, as shown in FIG. 7, a covering member 51 which has a plate 50 located more distally than the distal end face 7a of the distal-end constitution portion 7 and which can move freely in the axial direction G1, two hole portions 52 which are formed on the plate 50 and which respectively hold the distal sides of the first and the second arm portions 5A, 5B when the covering member 51 moves to the distal side, and the observation body 14 which is attached to the plate 50 and by which the frontal view is observed, are provided.

The endoscopic apparatus 53 constituted as above, as shown in FIG. 7, is inserted into the body cavity of the examinee in a state such that the covering member 51 is moved to the distal side and the distal sides of the first and the second arm portions 5A, 5B are held respectively by the two hole portions 52. In addition, the inserting portion 3 is fixed in a state such that the distal end portions of the two arm portions 5A, 5B are directed toward the diseased portion, and the covering member 51 is moved to the proximal side as shown in FIG. 8. Thus, the observation body 14 and the two hole portions 52 formed in the plate 50 move to the proximal side, and the first and the second arm portions 5A, 5B protrude in the frontal direction from the distal end of the inserting portion 3.

As mentioned above, according to the endoscopic apparatus 53 of the present example, similar effects to the first example can be achieved.

Next, the third example of an endoscope apparatus shall be described, and portions that are common with the abovementioned first and second examples shall be designated by the same reference numbers, descriptions thereof shall be omitted, and only the differences shall be described.

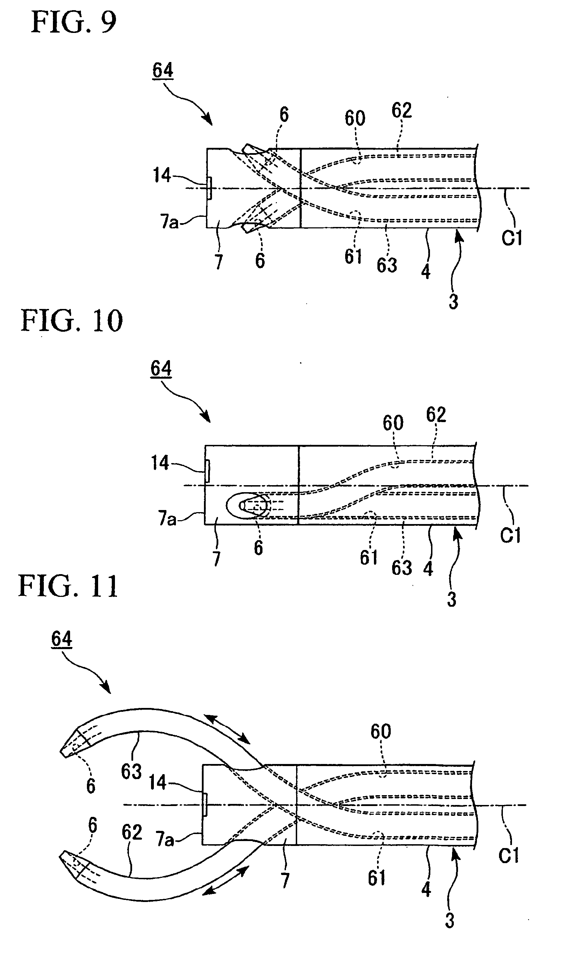

In this example, as shown in FIG. 9 and FIG. 10, two channels 60, 61 formed so as to open at the side faces of the distal end portion of the inserting portion 3 in front diagonal directions, and two bendable arm mechanisms 62, 63 inserted through the two channels 60, 61, respectively, in a freely retractable manner which are provided treatment tools 8A, 8B (not shown in these figures) inserted therein, the treatment tools 8A, 8B being capable of conducting a treatment, are provided. The distal side of each arm mechanisms 62, 63 can be bent by the not-illustrated operating wire provided therein.

The endoscopic apparatus 64 constituted as above, as shown in FIG. 9 and FIG. 10, is inserted into the body cavity of the examinee in a state such that the whole of the distal side of the two arm mechanisms 62, 63 is retracted in the two channels 60, 61 respectively. In addition, the inserting portion 3 is fixed in a state such that the distal end face 7a of the distal-end constitution portion 7 faces the diseased portion, and as shown in FIG. 11, a treatment is conducted by bending the distal end portion of each arm mechanism 62, 63 toward the axis C1 side of the inserting portion 3 while the two arm mechanisms 62, 63 are protruded to the distal side.

As mentioned above, according to the endoscopic apparatus 64 of the present example, the visibility in the frontal direction with the observation body 14 is increased when the inserting portion 3 is inserted into the body cavity and it is possible to prevent two arm mechanisms 62, 63 from damaging the peripheral tissue. In addition, the movable area of the treatment tools 8A, 8B which are inserted into the working channels 6 can be enlarged.

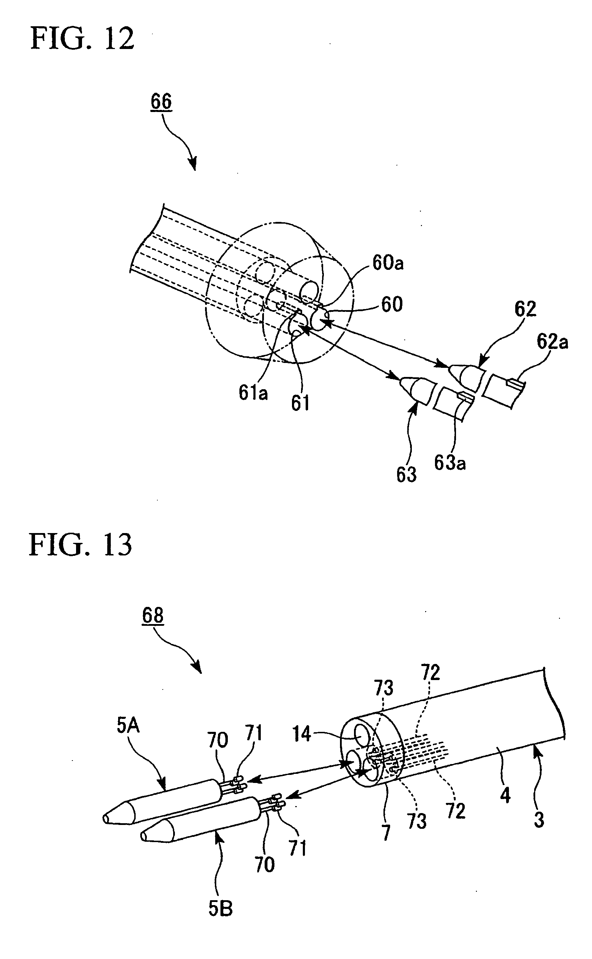

As shown in FIG 12, the arm mechanisms 62, 63 in the present example may be able to be removed from the endoscopic apparatus 66. At this time, ribs 62a, 63a may be formed in the arm mechanisms 62, 63 and concave portions 60a, 61a may be formed in the channel 60, 61 relative to the forms of the ribs 62a, 63a so that each orientation when inserting the arm mechanisms 62, 63 in the channels 60, 61 is always identical.

In addition, as shown in FIG. 13, the first and the second arm portions 5A, 5B may be constituted so as to be freely removed and attached to the distal-end constitution portion 7. The first connector 71 which is fixed to the proximal end portion of the first operating wire 70 for bending each arm portion 5A, 5B and the second connector 73 which is fixed to the distal end portion of the second operating wire 72 for pulling the first operating wire 70 enable the removal and attachment mentioned above. By the above constitution, an arbitrary length of the arm portion can be attached to the distal-end constitution portion 7, and the visibility in the frontal direction with the observation body 14 can be increased.

Next, the endoscope apparatus of the present invention shall be described, and portions that are common with the abovementioned first to third examples shall be designated by the same reference numbers, descriptions thereof shall be omitted, and only the differences shall be described.

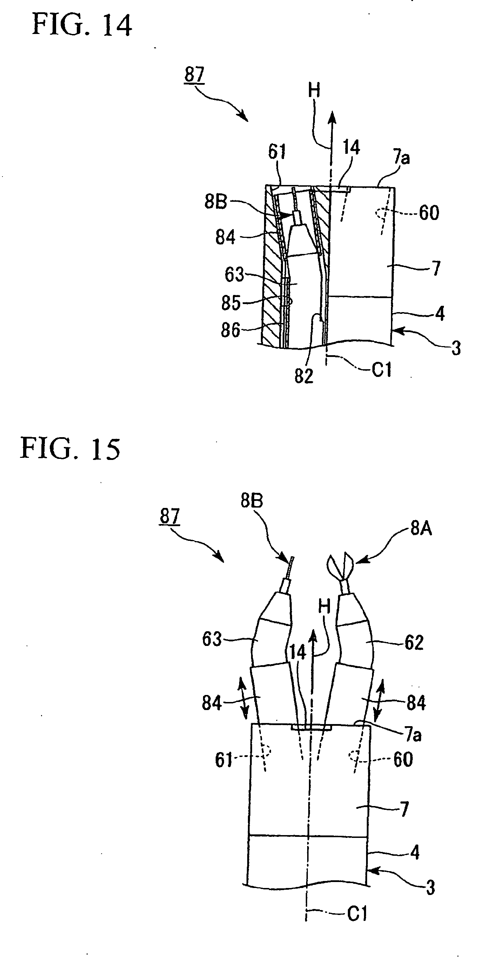

In this embodiment, as shown in FIG. 14, two channels 60, 61 formed in the distal end face 7a of the distal-end constitution portion 7; two bendable arm mechanism 62, 63 respectively inserted through the channels 60, 61 with free retraction and advancement which are provided with treatment tools 8A, 8B inserted therein, the treatment tools 8A, 8B being capable of conducting a treatment; an observation body 14 provided in the intermediate portion of the channels 60, 61 on the distal end face 7a of the distal-end constitution portion 7 by which the frontal view H is observed; and two cylindrical guide members 84 provided from the distal end portion of the channels 60, 61 with free retraction and advancement which lead the are mechanisms 62, 63 inserted therein to opposite oblique directions with respect to the frontal direction H of the observation body 14 , are provided.

The distal end portions of the channels 60, 61 are formed so as to tilt to the opposite oblique directions with respect to the frontal direction H of the observation body 14 respectively. In addition, the guide members 84 are made of a hard material, such as metal or the like, so that the portions of the arm mechanisms 62, 63 which are inserted into the guide members 84 become linear. The guide members 84 are provided to the distal sides of the channels 60, 61 respectively, and guide-member operating wires 86, which are inserted through the guide holes 85 formed in the channels 60, 61, are connected respectively to the proximal sides of the guide members 84. Furthermore, step portions 82 which engage the arm mechanisms 62, 63 with the proximal end portions of the guide members 84 are formed on the circumferential surface of the arm mechanisms 62, 63 with a predetermined distance from the distal end portion to the proximal side.

The endoscopic apparatus 87 constituted as above is inserted into the body cavity of the examinee in a state such that two arm mechanisms 62, 63 and the guide members 84 are not protruded in the frontal direction from the channels 60, 61 and retracted into the channels 60, 61 respectively by moving the two arm mechanisms 62, 63 to the proximal sides and pulling the guide-member operating wires 86 before being inserted into the body cavity. In addition, the inserting portion 3 is fixed and the two arm mechanisms 62, 63 are protruded to the distal side in a state such that the distal end face 7a of the distal-end constitution portion 7 faces the diseased portion, and thereby the guide members 84 are engaged with the step portions 82 which are formed in the arm mechanisms 62, 63, and protruded from the channels 60, 61 respectively. At this time, the guide members 84 support the arm mechanisms 62, 63 so that the proximal sides of the portions which are protruded from the channels 60, 61 in the arm mechanisms 62, 63 are directed to mutually opposite oblique directions with respect to the frontal direction of the observation body 14 and are not bent. Thus, the treatment is conducted by bending the distal end portion of each arm mechanism 62, 63 to the front H side of the observation body 14.

As mentioned above, according to the endoscopic apparatus 87 of the present embodiment, the visibility to the frontal direction H by using the observation body 14 is increased when the inserting portion 3 is inserted into the body cavity and it is possible to prevent the two arm mechanisms 62, 63 from damaging the peripheral tissue. In addition, the distal end portion of the each arm mechanism 62, 63 can be observed without being interfered by the proximal side of each arm mechanism 62, 63 when the treatment is conducted.

Next, the the fourth example of an endoscope apparatus shall be described, and portions that are common with the abovementioned first to third examples shall be designated by the same reference numbers, descriptions thereof shall be omitted, and only the differences shall be described.

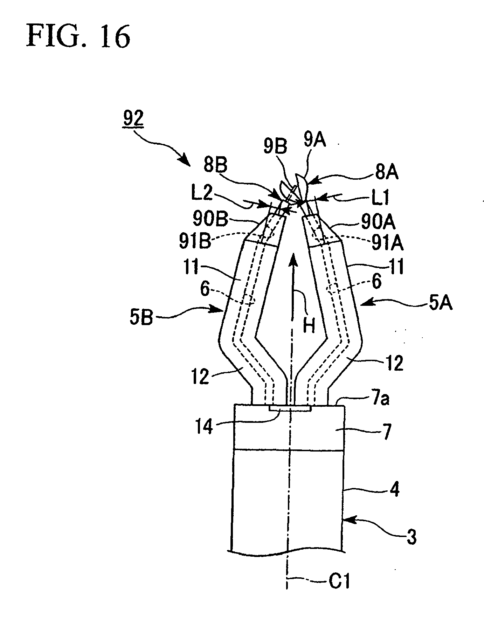

In this example, as shown in FIG. 16, an observation body 14 which is provided in the intermediate portion of the first and second arm portions 5A, 5B on the distal end face 7a of the distal-end constitution portion 7 by which the frontal direction H is observed, and bending guide members 90A, 90B provided in the distal end portions of the arm portions 5A, 5B respectively which bend the treatment tools 8A, 8B so that the distal ends of the protruded treatment tools 8A, 8B are positioned to the front H side of the observation body 14, are provided.

Each bending guide member 90, 91 is made of a hard material, such as metal or the like, and each working channel 91A, 91B is fonned respectively inside thereof. Each treatment tool 8A, 8B is inserted through each working channel 91A, 91B and each bending guide member 90, 91 communicates with each working channel 6 formed in each arm portion 5A, 5B.

In this example, the distal end portion of the working channel 91A or the bending guide member 90A is eccentric to the axis C1 side by the distance L1 relative to the axis of the working channel 6 in the distal end portion of the first arm portion 5A. In the same way, the distal end portion of the working channel 91B of the bending guide member 90B is eccentric to the axis C1 side by the distance L2 relative to the axis of the working channel 6 in the distal end portion of the second arm portion 5B.

As mentioned above, according to the endoscopic apparatus 92 of the present example, it is possible to observe the distal portion of each arm portion 5A, 5B and treatment portion 9A, 9B without being interfered by the proximal side of each are portion 5A, 5B when the treatment is conducted.

In addition, the first modification of the fourth example shall be described, and portions that are common with the abovementioned fifth example shall be designated by the same reference numbers, descriptions thereof shall be omitted, and only the differences shall be described.

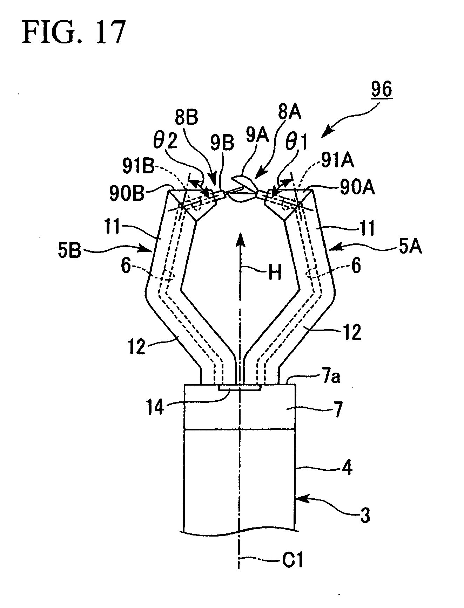

As shown in FIG. 17, in this modification, the working channel 9 1A of the bending guide member 90A is tilted to the from H side of the observation body 14 by the angle 91 relative to the axis of the working channel 6 in the distal end portion of the first arm portion 5A. In the same way, the working channel 91B of the bending guide member 90B is tilted to the front H side of the observation body 14 by the angle θ2 relative to the axis of the working channel 6 in the distal end portion of the second arm portion 5B.

As mentioned above, according to the endoscopic apparatus 96 of the present modification, the similar effects to the fourth example mentioned above can be achieved.

Furthermore, the second modification of the fourth example shall be described, and portions that are common with the abovementioned firth example shall be designated by the same reference numbers, descriptions thereof shall be omitted, and only the differences shall be described.

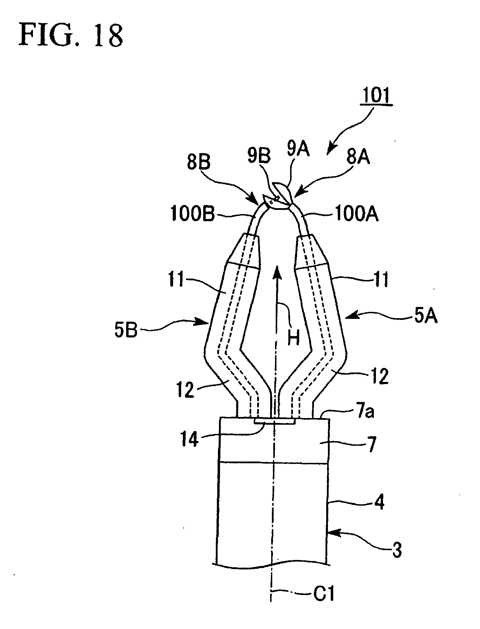

As shown in FIG. 18, in this modification, bending-inclination portions 100A, 140B are formed in the treatment tools 8A, 8B respectively so as to be bent to the front H side of the observation body 14 respectively. The method to form the beading-inclination portions 100A, 100B is to provide a heat treatment to the sheath with which the circumferential surface is covered, to shape and transform the sheath, or the like before the assembly of the treatment tools 8A, 8B or after the assembly of the treatment tools 8A, 8B.

As mentioned above, according to the endoscopic apparatus 101 of the present modification, the similar effects to the fourth example mentioned above can be achieved.

Some of the above has been a description of a preferred embodiment of the present invention, but the present invention is not limited to such working examples. Additions, omissions, substitutions, and other modification can be made to the configuration within a scope that does not depart from the claims.

For example, in the above-described first example, the case was described where moving the sheath 4 and the observation body 14 to the axial direction G1 in unity or moving only the sheath 4 is selectable by changing the depth to be pushed of the operating lever 15, but the sheath 4 and the observation body 14 may always be moved to the axial direction G1 in unity.

In addition, in the above-described first and second examples, the case was described where two arm portions which are the first and the second arm portions 5A, 5B are provided on the distal end face 7h of the distal-end constitution portion 7, but three or more arm portions may be provided. In the above-mentioned third example and its modifications, the case was described where two arm mechanisms 62, 63 are provided, but three or more arm mechanisms may be provided.

The present invention is not limited by the foregoing descriptions, and is only limited by the scope of the appended claims.

## Claims

1. An endoscopic apparatus (87) comprising:
an elongate tubular inserting portion (3);
a plurality of arm portions (62, 63) protruding from a distal end face (7a) of the inserting portion (3) in a frontal direction, wherein the arm portions (62, 63) are provided with treatment tools (8A, 8B) inserted therein and are inserted in a cylindrical guide member (84) so as to freely retract and advance, the treatment tools (8A, 8B) being capable of conducting a treatment; and
an observation body (14) by which a frontal view is observed,
**characterised in that** the endoscopic apparatus (87) further comprises a cylindrical sheath (4) which covers a circumferential surface of the inserting portion (3), which is movable to a distal side and a proximal side, and which supports the distal side of the plurality of the arm portions (5A, 5B) when moving to the distal side, wherein the observation body (14) is attached inside a distal end portion of the sheath (14), and wherein each cylindrical guide member (84) is provided at a distal end portion of the inserting portion (3) so as to freely retract and advance, wherein the cylindrical guide members (84) are adapted to lead the arm portions (62, 63) inserted therein to opposite oblique directions respectively with respect to the frontal direction of the observation body (14) and each of the arm portions (62, 63) has a step portion (82) which engages with a proximal end portion of the guide member (84).

2. The endoscopic apparatus (87) of claim 1, wherein:
the observation body (14) is provided in an intermediate portion of two channels (60, 61) receiving the arm portions (62, 63) on the distal end face (7a) of the inserting portion (3).

## Patentansprüche

1. Endoskopvorrichtung (87) mit:
einem langgestreckten rohrförmigen Einführabschnitt (3);
einer Mehrzahl von Armabschnitten (62, 63), die von einer distalen Endfläche (7a) des Einführabschnitts (3) in einer frontalen Richtung vorstehen, wobei die Armabschnitte (62, 63) mit darin eingeführten Behandlungsinstrumenten (8A, 8B) versehen sind und in ein zylindrisches Führungselement (84) eingeführt sind, um sich frei zurück und nach vorne zu bewegen, und wobei die Behandlungsinstrumente (8A, 8B) dazu eingerichtet sind, eine Behandlung durchzuführen; und
einem Beobachtungskörper (14), durch den eine Frontalansicht beobachtet wird,
**dadurch gekennzeichnet, dass** die Endoskopvorrichtung (87) ferner eine zylindrische Hülse (4) umfasst, die eine Umfangsfläche des Einführabschnitts (3) bedeckt, die zu einer distalen Seite und einer proximalen Seite bewegbar ist und die die distale Seite der Mehrzahl von Armabschnitten (5A, 5B) abstützt, wenn sie sich zu der distalen Seite bewegt, wobei der Beobachtungskörper (14) im Inneren eines distalen Endabschnitts der Hülse (14) befestigt ist, und wobei jedes zylindrische Führungselement (84) an einem distalen Endabschnitt des Einführabschnitts (3) vorgesehen ist, um sich frei zurück und nach vorne zu bewegen, wobei die zylindrischen Führungselemente (84) dazu eingerichtet sind, die darin eingeführten Armabschnitte (62, 63) jeweils bezüglich der frontalen Richtung des Beobachtungskörpers (14) in entgegengesetzte schräge Richtungen zu führen und jeder der Armabschnitte (62, 63) einen Stufenabschnitt (82) hat, der mit einem proximalen Endabschnitt des Führungselements (84) zusammenwirkt.

2. Endoskopvorrichtung (87) nach Anspruch 1, wobei:
der Beobachtungskörper (14) in einem mittleren Abschnitt von zwei Kanälen (60, 61) vorgesehen ist, die die Armabschnitte (62, 63) an der distalen Endfläche (7a) des Einführabschnitts (3) aufnehmen.

## Revendications

1. Appareil d'endoscope (87) comprenant :
une partie d'insertion tubulaire allongée (3) ;
une pluralité de parties de bras (60, 63) faisant saillie depuis une face d'extrémité distale (7a) de la partie d'insertion (3) dans une direction frontale, dans lequel les parties de bras (62, 63) sont munies d'outils de traitement (8A, 8B) insérés dans celles-ci et sont insérées dans un élément de guidage cylindrique (84) de façon à reculer et avancer librement, les outils de traitement (8A, 8B) étant capables de réaliser un traitement ; et
un corps d'observation (14) par lequel une vue frontale est observée,
**caractérisé en ce que** l'appareil d'endoscope (87) comprend en outre une gaine cylindrique (4) qui couvre une surface circonférentielle de la partie d'insertion (3), qui peut être déplacée vers un côté distal et un côté proximal, et qui supporte le côté distal de la pluralité des parties de bras (5A, 5B) lors du déplacement vers le côté distal, dans lequel le corps d'observation (14) est fixé à l'intérieur d'une partie d'extrémité distale de la gaine (14), et dans lequel chaque élément de guidage cylindrique (84) est prévu au niveau d'une partie d'extrémité distale de la partie d'insertion (3) de façon à reculer et avancer librement, dans lequel
les éléments de guidage cylindriques (84) sont adaptés pour mener les parties de bras (62, 63) insérées dans ceux-ci vers des directions obliques opposées respectivement par rapport à la direction frontale du corps d'observation (14), et chacune des parties de bras (62, 63) comporte une partie étagée (82) qui s'engage avec une partie d'extrémité proximale de l'élément de guidage (84).

2. Appareil d'endoscope (87) selon la revendication 1, dans lequel :
le corps d'observation (14) est prévu dans une partie intermédiaire de deux canaux (60, 61) recevant les parties de bras (62, 63) sur la face d'extrémité distale (7a) de la partie d'insertion (3).
